# EUROPEAN PATENT APPLICATION

(11) **EP 4 245 860 A1**
(43) Date of publication of application: **20.09.2023**
(21) Application number: 20956912.8
(22) Date of filing: 17.11.2020
(51) Int. Cl.: C12Q 1/6806, B01D 15/08

(54) **DEVICE FOR NUCLEIC ACID EXTRACTION AND PURIFICATION**

(30) Priority: 12.10.2020 CL 20202629
(71) Applicant: Taag Genetics Corp, St. Charles, IL 60174 (US)
(72) Inventor: BERNDT BRICEÑO, Denis Gustavo, Santiago, 9031254 (CL); MALIG FUENTES, Rodrigo Fernando, Santiago, 9031254 (CL); NIKLITSCHEK OYARZÚN, Mauricio Alejandro, Santiago, 9031254 (CL)
(74) Representative: Elzaburu S.L.P.
(86) International application number: PCT/CL2020/050157
(87) International publication number: WO 2022/077128

(57) **Abstract**

The present invention describes a new device for extracting and purifying nucleic acids from a biological sample, which comprises: a closed container that holds a solid phase comprising a mixture of non-ionic resins, preferably aromatic resins, combined with a buffering solution or water, or the sample alone. The invention also describes a method for detecting a nucleic acid, comprising the following steps: (1) providing a sample of biological origin; (2) depositing the sample of biological origin containing the nucleic acids in the closed container; (3) heating the sample; (4) taking a volume of the sample; and (5) detecting the presence of the nucleic acid of interest using PCR or any variant thereof.

## Description

### FIELD OF THE INVENTION

The present invention is part of the field of purification of nucleic acids from different complex biological samples, which have various inhibitors, making it difficult to obtain nucleic acids in quality and quantity to be used in different molecular biology techniques. In particular, the present invention describes new molecular biology devices that comprise adsorption resins, which facilitate the extraction of nucleic acids by reducing purification steps from different types of samples. Furthermore, the present invention thereby simplifies the extraction and purification of nucleic acids, allowing anyone with minimal training in molecular biology to perform the extraction process in a single step.

### BACKGROUND OF THE INVENTION

**Advances** in the technical field of molecular biology, and in particular the advance in the detection of nucleic acids by PCR and its different variants, have revolutionized the detection of pathogenic or non-pathogenic microorganisms in industrial sectors such as food, clinical, treatment water, agriculture, mining, among others. In particular, the timely detection of pathogenic microorganisms in different types of food, or food matrices, and also in different clinical samples, poses a series of challenges for companies that offer detection services or that market reagents for this purpose.

The main challenge in this technical field is to develop a nucleic acid extraction and purification product that is universal, that is, that is useful for efficiently extracting and purifying nucleic acids in different types of samples from each industry. This is a huge problem that has no current solution because each sample, having a different composition, can present different technical challenges in itself.

The nucleic acid extraction process generally follows the following steps:
1. Cell rupture;
2. Elimination of cell debris, proteins, carbohydrates, lipids and other molecules; and
3. Obtain a solution enriched with nucleic acids.

**The** state of the art teaches that there are, mainly, two methods used to perform cell disruption: mechanical disruption, chemical disruption, and enzymatic disruption. Mechanical rupture is based on cell rupture using 0.5 mm "glass balls" which, when added to a biological sample and vortexed, release the nucleic acids contained in the cells present in the biological sample. Instead, enzymatic breakdown relies on the differential digestion of the plasma membranes of different microorganisms. Traditionally, lysozyme (alone or in combination with other agents) is used to disrupt bacterial cells and chitinase is used for yeast and fungal cells. However, as described in various articles (see for example: Ohta A., et al. A Review on Macroscale and Microscale Cell Lysis Methods. Micromachines (Basel). 2017 Mar; 8(3):83) to extract and purify nucleic acids from some microorganisms, it is necessary to use a combination of mechanical and enzymatic (or other) methods to obtain quality and quantity nucleic acids from different biological samples. Additionally, the use of enzymes in cell disruption increases the extraction costs per reaction. Consequently, enzymatic breakdown methods are widely used by laboratories or research centers (which process few samples), while the use of enzymes is avoided in private laboratories that routinely perform this type of analysis.

The next step after cell disruption is the separation of the cellular components present in the cell lysate (proteins, lipids, carbohydrates, and various other molecules) from the nucleic acids. To carry out this task, the use of solid phase extraction (SPE) nucleic acid purification methods has been developed and widespread. SPE is based on liquid and stationary phases, which selectively separate the target analyte from solution based on the specific hydrophobic, polar, and/or ionic properties of both the solute and the sorbent. The chemistry between the sorbent and the analyte of interest is the basis of this technique, while "weak" chemical interactions such as van der Waals forces (nonpolar interactions), dipole-dipole interactions (polar interactions), and bonds of hydrogen determine the retention mechanism in SPE.

SPE methods can be divided into normal/regular SPE, reverse SPE, and ion exchange SPE. Each sorbent used in SPE has unique characteristics, resulting in a solution to a specific problem involved in extraction methods.

Regardless of the type of SPE, different resins have been developed that are compatible with the various formats that an SPE purification process can take. The most commonly used resins or matrices are those based on silica, glass, diatomaceous earth, magnetic beads, anion exchange materials, cellulose matrices, methacrylates and different organic and inorganic polymers. Of all these matrices, the most used have been those based on silica due to their well-known binding properties to nucleic acids. In recent times, polymeric matrices have emerged in various purification processes because they are cost-effective, and also have improved properties compared to other materials.

One of the polymer matrices that has gained the most attention are those derived from the polydivinylbenzene copolymer. Said polymer is produced from the polymerization of divinylbenzene with styrene, and due to the presence of large aromatic groups in its structure, it has a hydrophobic capture behavior. Specifically, this type of resins is defined as non-ionic since they do not have ionizable functional groups in their structure, and they are resistant in pH ranges ranging from 0 to 14. However, this type of resins can be modified by coupling different (ionizable) functional groups and thus a non-ionic resin can be adapted for different types of chromatography. This versatility of this type of hydrophobic resin has made it one of the most widely used in chromatographic processes, as it is capable of maintaining its properties over a wide pH range.

As one skilled in the art will recognize, although there are numerous types of resins, the most commonly used currently for nucleic acid purification are ion exchange resins, and specifically anion exchange resins, due to their affinity for negative molecules such as nucleic acids. Document WO2013144654A1, which is incorporated by reference in its entirety, summarizes the state of the art of resins that have affinity for nucleic acids.

However, the purification of nucleic acids by ion exchange chromatography generates some problems in the subsequent PCR steps since there are negatively charged molecules that co-purify with the nucleic acids bound to the resins, and that can act as inhibitors.

Considering all of this background information, it is evident that there is an unsatisfied problem in the art, which corresponds to generating a nucleic acid extraction and purification device that is easy to perform, that is compatible with numerous matrices, and that is competitive in in terms of its marketing cost.

In order to better describe the present invention, a prior art search was performed, and nearby documents found are summarized below.

US2019100788A1 describes a technology related to the isolation of nucleic acids. In particular, the technology relates to methods and kits for extracting nucleic acids from problematic samples such as feces. Claim 8 describes a method for removing a PCR inhibitor from a crude sample preparation comprising a nucleic acid, wherein the method comprises: a) adding insoluble polyvinylpyrrolidone to said crude sample preparation prior to isolating the nucleic acid in conditions under which said test inhibitor binds said polyvinylpyrrolidone to produce a complex; b) separating the crude sample preparation complex to produce a clarified sample preparation including said nucleic acid.

US2017152501A1 describes a process for the purification and/or isolation of nucleic acids where the process comprises: (1) a cell lysis, which produces a sample that contains nucleic acids and proteins, (2) contacting the sample with a sorbent material that binding the proteins, and collecting the eluate containing the nucleic acids, wherein the sorbent material comprises a porous inorganic material comprising silica that is at least partially covered by a polymer.

US2014363819A1 describes compositions and methods for improving the amplification or detection of a target nucleic acid in a sample that contains PCR inhibitors, such as polyphenols. An enhancer composition is provided that includes casein or polyvinylpyrrolidone, or a modified polymer thereof.

WO2013144654A1 describes a method for passing a liquid sample through a porous solid matrix, comprising the following steps: (1) sealing the liquid sample inside a container comprising a porous solid matrix or at least a part of the container and (2) raise the temperature to increase the pressure inside the container, therefore causing the liquid to pass through the porous solid matrix. In claim 4 it is described that nucleic acids have affinity for the matrix while inhibitors leave in the eluate.

WO2004020971A2 describes a method for preparing adenovirus particles from an adenovirus preparation comprising the steps of: (a) subjecting said adenovirus preparation to chromatography on a first chromatographic medium, whereby adenovirus particles from said adenovirus preparation are retained on said first chromatographic medium; (b) eluting adenovirus particles from said first chromatographic medium to produce an adenovirus particle eluate ; (c) subjecting adenovirus particles from said eluate to chromatography on a second chromatographic medium, wherein said second chromatographic medium retains one or more contaminants from said eluate and wherein said second chromatographic medium is not solely a size exclusion medium ; and (d) collecting adenovirus particles from said eluate . The specification of claims describes that the second chromatographic medium is BioSepra Blue Trisacryl resin. This is a nonionic resin that separates by hydrophobic interactions.

DE19731670A1 describes the purification of nucleic acids from biological samples, which comprises treating the sample with a synthetic anion exchange resin having a binding affinity for bile acids so that any inhibitors of the subsequent analytical reaction are bound to the resin and removed. The use of cholestyramine (polystyrene cross-linked with divinylbenzene with quaternary ammonium groups) or colestipol (diethylenetriamine-epichlorohydrin copolymer) for purification and/or isolation from biological samples is also claimed .

"Current nuclear Acid extraction Methods and Their Implications to Point- of - Care Diagnostics". Ali Nasir et al. 2017.BioMed _ Research International Volume 2017, Article ID 9306564". describes Chelex as a styrene- divinylbenzene copolymer that has iminodiacetate ions covalently attached to it, which are used as chelators of polyvalent metal ions. Chelex is described as an interesting technique as it is fast, has few steps, and does not use dangerous chemicals such as phenol/chloroform. Its main drawback is the inability to efficiently remove PCR inhibitors from complex samples due to the lack of purification steps.

Taking this into consideration, few documents were found in which a nucleic acid purification method is disclosed that involves a resin that does not bind nucleic acids but is capable of retaining the inhibitors present in the sample.

It should be noted that the guiding principle of the present invention is based on cell lysis and molecular denaturation by temperature, where in this same stage a solid matrix preferably composed of resins with aromatic groups captures the inhibitors and the nucleic acid remains free of inhibitors for its use. subsequent analysis. This purification principle is just contrary to all the products and methods described in the prior state of the art, and they cannot be derived in any way from the combination of different documents available in the prior art at the time of presentation of the present invention. Heat treatment of biological samples that allows cell lysis and molecular denaturation in the presence of an aromatic nonionic resin allows sensitive and direct detection of nucleic acids without the need for slow and complex purification steps.

### SUMMARY OF THE INVENTION

The present invention describes a new device for the extraction and purification of nucleic acids from a biological sample, comprising: a closed container containing a solid phase comprising a mixture of nonionic resins, preferably aromatic, in combination with a buffer solution or water or just the sample. The invention further describes a method of detecting a nucleic acid comprising the following steps: (1) providing a sample of biological origin, (2) depositing the sample of biological origin containing the nucleic acids in the closed container, (3) heating the sample, (4) taking a volume of the sample, and (5) detecting the presence of the nucleic acid of interest by PCR or any of its variants.

### DETAILED DESCRIPTION OF THE INVENTION.

As previously indicated, the present invention corresponds to a device for the extraction and purification of nucleic acids from a biological sample, comprising: a closed container containing a solid matrix comprising a mixture of solid nonionic resins, preferably aromatic and optionally a buffer solution. In order to exemplify the invention and show the main elements that compose it, the description of the same will be made using a particular case, and in the examples section, 7 different embodiments of the invention are shown.

The choice of the particular embodiment corresponding to a device for the extraction and purification of the nucleic acids of the SARS-COV-2 virus present in a human biological sample, is due to the need to detect the presence of this pandemic virus in a timely and precise manner. in the world as it is a global health problem.

The SARS-COV-2 virus is a betacoronavirus and is the agent responsible for coronavirus disease 2019 (COVID-2019). The picture generated by this virus is a severe acute respiratory syndrome and was identified as a pandemic by the World Health Organization (WHO) on March 11, 2020. To date, this new virus has infected 37 million people and caused the death of around 1 million of them. This is an enveloped, non-segmented, positive-sense RNA virus, its diameter is about 65-125 nm, it contains single-stranded RNA, and it is provided with crown-shaped spikes on the outer surface. In this SARS-CoV-2 pandemic, reliable, early and accurate diagnosis is crucial to provide timely medical help to the infected person, as well as help government agencies prevent its spread to others and save lives. False negative test results can lead to the spread of the epidemic in the community. Similarly, a false positive result can lead to unnecessary treatment and mental trauma for patients.

One of the main causes of the delay in the delivery of tests that seek the detection of the virus is the processing of the samples (extraction and purification of nucleic acids). Currently available detection kits such as the EZNA^{®} Total RNA Kit I from Omega Bioteck (catalog number: R6834-02 x 200 rx, or Cat. R6834-01 x 50 rx) are complex in terms of their use as they have a series steps and solutions to be added sequentially. The complexity of the protocol, in addition to consuming man-hours for the technical staff that performs the processing of the samples, can generate involuntary errors in the processing of the samples to be extracted.

For these reasons, the extraction and purification of SARS-COV-2 nucleic acids from human biological samples in a simple and robust manner is still a permanent unmet need in the art.

A particular solution to the problem of detecting SARS-COV-2 corresponds to analyzing biological samples from nasopharyngeal swabs from human patients who are presumably infected with the virus, applying the aforementioned nucleic acid extraction and purification device, which comprises a closed container containing a solid matrix comprising a solid nonionic resin or a mixture of said resins and optionally a buffer solution.

Divinylbenzene group with a macropore structure and having a large surface area. Among the resins derived from styrene-divinylbenzene, the most preferred resins to be used in the invention can be selected from the following: PuroSorb^{®} PAD400, PAD500, PAD600, PAD900, PAD1200, Amberlite ^{®} FPX66, FPX68, Amberlite^{®} XAD2, XAD4, XAD16, XAD1180, XAD200, XAD2010, Diaion^{®} and Sepabeads^{®} HP20, HP20SS, HP21, SP70, SP700, SP825L, SP850, CHP20, CHP50, SP207, LEWATITO AF 5, SEPLlTE^{®} CT10, LX20, LX 207, LXA8, LXA10 , LXA17, LXA680, LXA1600, LXA1180, LXA81, LXA816, LXA817, LXA8302, LXA88, LXS868, AB-8. This is a list that should not be taken as limiting for the present invention, but are only examples of aromatic nonionic resins that can be used as technical equivalents of the present invention.

A person skilled in the art of the invention will recognize that the novel nucleic acid extraction and purification principle described in the present invention is based on the unexpected properties of hydrophobic resins, which retain PCR inhibitors present in different types of samples. Therefore, any nonionic, hydrophobic resin could act as an adsorbent for said inhibitors, and the particular description of the resins named above should not be taken as limiting the present invention.

In relation to the choice of the buffer solution in which the mixture of solid resins is embedded, it must be taken into account mainly that the buffer solution is capable of preserving the state of the biological sample, for example, during its transport, and not it has to do with a choice related to the resin itself. As previously mentioned, the resins of the present invention are stable over the entire pH range and do not change their adsorption properties. For the specific embodiment related to the detection of SARS-COV-2 from human swab samples of various types, the buffer is 1X PBS pH 8.0.

However, said particular exemplification of a buffer solution should not be taken as limiting the scope of the device of the present invention. If it is desired to detect the presence of nucleic acids in a biological sample that is preserved in acidic pH ranges, the solid matrix containing the resin should be embedded in a buffer solution in said pH range. The same applies to a biological sample that must be kept at a basic pH. However, there are different biological samples that do not use a buffer solution for processing, but the sample can be added directly to the container containing the resin without the need to buffer the pH of the solution.

The buffer solution of the present invention can be selected from the following buffers: citric acid, phosphate, MES, Bis-Tris, ADA, ACES, PIPES, MOPSO, Bis-Tris Propane, BES, MOPS, TES, HEPES, DIPSO, TAPSO, Tris, HEPPSO, POPSO, TEA, EPPS, Tricine , Gly-Gly , Bicine , HEPBS, TAPS, AMPD, TABS, AMPSO, CHES, CAPSO, AMP, CAPS and/or CABS.

The present invention also relates to a method of preparing the solid matrix that comprises massing suitable amounts of one or several non-ionic adsorption resins, depositing them in a suitable container and mixing them with a suitable amount of a buffer solution.

In a preferred embodiment the resin:buffer ratio is 0.6 grams resin:1 mL buffer. Said resin:buffer solution mixture can be deposited in a container of a suitable size to contain said solution.

In another slightly more specific embodiment, the proportion of resin is 0.06 grams with 100 µL of sample without buffer. It is important to note that the sample does not necessarily have to be deposited directly on the resin. Depending on the type of sample, for example, a spiked food sample, the sample may be premixed with a buffer solution and then a volume of said mixture may be heat treated in the presence of the nonionic resin.

Although in one exemplification of the invention, the nonionic resin of the invention is present as a free solid matrix in a container, said matrix could also be used in other formats such as embedded in filters that can be incorporated into containers such as eppendorf tubes or other suitable plastic tubes. The present invention should not be thought of as limiting its use as described in the particular examples, but it could additionally be used in HPLC-type purification systems or suitable chromatographic systems.

A container in the context of the present invention is understood as any molecular biology grade container that can contain reagents of suitable purity. These containers can be, but should not be limited to the following: 50 mLor 15 mL Falcon type tubes, Eppendorf tubes in all their formats (2 mL, 1.5 mL, 1 mL , 0.5 mL), PCR tubes in all its formats, 96-well plates compatible with PCR systems and 384-well plates. Each of said containers could be used to carry out the nucleic acid extraction and purification process.

The invention further describes a method of detecting a nucleic acid comprising the following steps:
1. take a sample of biological origin,
2. deposit the sample of biological origin containing the nucleic acids in the closed container,
3. heat the sample,
4. take a volume of the sample, and
5. Detect the presence of the nucleic acid of interest by means of PCR or any of its variants.

In a preferred embodiment, the biological sample can have different origins. Biological samples can be obtained from environmental samples, from production processes, clinics and/or from various surfaces. Environmental samples may correspond to liquid or solid samples, or mixtures thereof (mud). Samples of production processes can be taken from the agricultural, food, fruit, mining, metallurgical, and dairy industries, among others. The agricultural samples may correspond to a part of the vegetables that are being cultivated, and in the same way the fruit samples may correspond to portions of fruits in the pre-harvest stage, during their processing, packaging or post-harvest. Samples from the food industry can be ready-to-eat foods and other types of processed or pre-processed foods.

In another preferred embodiment, the biological samples to be detected contain animal or plant cells, bacteria or viral particles whose nucleic acids are to be detected and inhibitors that interfere with the detection of said nucleic acids.

In an even more specific preferred embodiment, the samples may contain inhibitory substances of the polymerase chain reaction, in all its variants. PCR inhibitors can be of different types, but the most common are: humic acid found in plants and soil, polyphenols, certain divalent metals, collagen, and pigments. Additionally, the matrices that frequently present this type of inhibitors are selected from chocolates, coffee, samples that have dyes (berries) and some spices.

The invention allows the removal of inhibitors that are normally found forming part of complex molecular structures within the sample of biological origin. These molecular structures are affected by the heat treatment in such a way that the hydrophobic chemical groups are exposed and can be absorbed by the resin present in the device described in the present invention. Surprisingly, the adsorption of the inhibitors on the non-ionic resin is such that it allows the direct detection of the nucleic acids from the extraction of a portion of the supernatant liquid inside the device, without the need for additional purification steps.

In relation to the sampling device, this can be different types. For clinical samples, a sterile swab can be used and after the sample is taken from the patient, it must be deposited in the aforementioned device and the device must be closed during transport. In relation to the taking of clinical samples, these can be taken from different parts of the human body: skin, mucous membranes, hair, nails and fluids.

In another additional preferred embodiment, the clinical samples may correspond to nasopharyngeal swabs, oropharyngeal swabs, nasal swabs, oral swabs, vaginal swabs, cervical swabs, urethral swabs, saliva, and dermatological samples.

In another preferred embodiment, the sample taken from different environments may require a treatment prior to the extraction and purification of nucleic acids by the device of the invention. For example, this occurs when analyzing food samples where said samples must be incubated in an enrichment medium for a period of time in order to increase the number of microorganisms present in the sample. In these cases, the sample can be taken and does not need to be deposited immediately in the device of the invention.

In relation to sample heating, this is defined as the process of incubating the device with the sample inoculated inside at a temperature between 50ºC and 100ºC for a period of at least 1 to 30 minutes, preferably at 95ºC for at least less 15 min. It is worth mentioning that said heat treatment must be such that it allows cell lysis and molecular denaturation, which in turn allows the adsorption of the inhibitors on the resin present during the heat treatment. As an expert in the field will recognize, biological samples can be treated with different combinations of temperatures and time, all of which can achieve the same effect of cell lysis and molecular denaturation, which in turn allows adsorption of the inhibitors on the resin present during heat treatment.

Other novel characteristics that cannot be derived from the state of the art is that the entire nucleic acid extraction and purification protocol is carried out in the same container, and does not require centrifugation steps.

Therefore, after incubating the sample at a suitable temperature and for an appropriate period of time, a volume of the purified nucleic acid sample is taken and used directly as a template for PCR reactions, or any technique that requires nucleic acids such as isothermal amplification, DNA sequencing, among others.

The PCR variants by which a nucleic acid can be detected can be the following: real-time PCR, RT-PCR, Multiplex PCR, nested PCR, Hot start PCR, among others. With respect to the real-time PCR technique, a person normally skilled in the art understands that said technique employs different fluorophores to detect the presence of the target nucleic acid to be identified. There are different fluorophores that can be excited with specific wavelengths, and that also emit in a specific wavelength range.

The commonly used fluorophores are FAM, ROX and HEX, but in fact any other fluorophore available in the state of the art that has been used in real-time PCR is compatible with the detection method of the present invention.

In a real-time PCR assay, a positive reaction is detected by the accumulation of a fluorescent signal. The Ct (Cycle Threshold) is defined as the number of cycles required for the fluorescent signal to cross the threshold (ie, exceed the background level). In the context of the present invention, a real-time PCR reaction is more sensitive than another, if it has a Ct of at least 0.5 difference compared to another reaction, preferably greater than 1.0.

### FIGURE DESCRIPTION

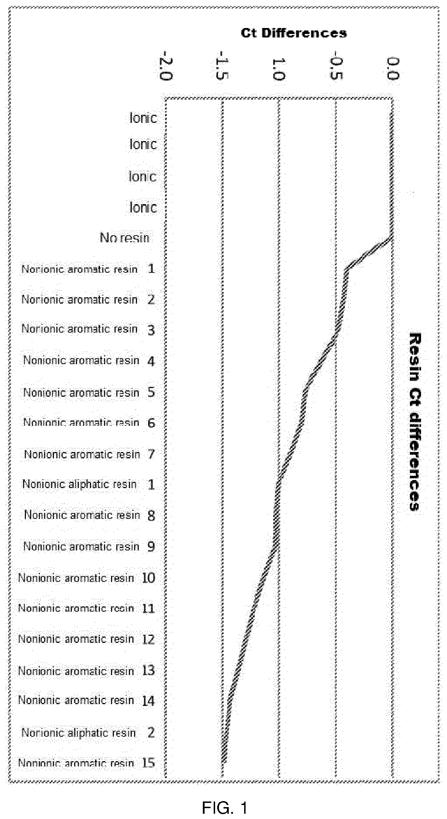

Figure 1 show efficiency of different types of resin for the detection of SARS-COV2 compared to the result obtained from the sample without resin. A negative result means that the new Ct is lower (higher sensitivity) than the Ct obtained from the sample without resin.

### EXAMPLES

### Example 1: Comparison of extraction and purification of samples from nasopharyngeal and oropharyngeal swabs using the device of the invention versus other resins.

During the development of the present invention, the first thing that was evaluated was to determine what type of resin was the one that allowed extracting and purifying SARS-COV-2 RNA from human clinical samples. All the resins had a polystyrene polymer or a styrene- divinylbenzene copolymer as their skeleton, but they differed in that some of these resins had other functional groups covalently attached, which allowed them to have charges at different pHs.

In this preferred exemplification of the invention, the presence of SARS-COV-2 was detected by amplifying the N1 gene, which codes for the viral nucleocapsid and is one of the most widely used markers to determine the detection of coronaviruses in human clinical samples. Additionally, the gene that codes for human RNAase P (RP) was detected, which corresponds to the test sample control.

As previously mentioned, one of the ways to determine the sensitivity of different detection methods through real-time PCR is to determine the Ct with which the sample is classified as positive. A lower Ct in a given treatment indicates that the method is more sensitive in said condition, and this is what is always sought when working with real-time PCR systems.

The extraction protocol used in this example comprises the following steps:
1. take a biological sample positive for SARS-CoV-2;
2. Deposit 100 µL of the sample of biological origin containing the nucleic acids in different containers each with 60 mg of different resins;
3. heat the sample for 15 min at 95°C;
4. Take 5 µL of the sample, and
5. Detect the presence of the SARS-CoV-2 N1 gene by real-time RT-PCR.

The resins evaluated and the results obtained are shown in Table 1.

**Table 1. Effect of the type of resin on the detection of SARS-COV-2.**

| **Number** | **Condition** | **Type of resin** | **Ct N1** |
|---|---|---|---|
| 1 | Boiled sample without resin | NA | 33.3 |
| 3 | SEPLITE^{™} MB20 | mixed bed exchange | 37.6 |
| 4 | SEPLITE^{™} LSC660 | ion exchange | 34.0 |
| 5 | SEPLITE^{™} LSC720 | ion exchange | 34.4 |
| 6 | SEPLITE^{™} LSC724 | ion exchange | 35.7 |
| 7 | AmberLite^{™} FPX66 | adsorption | 32.1 |
| 8 | Purosorb^{™} PAD900 | adsorption | 32.0 |

As can be seen in Table 1 of this application, the methods with the adsorption resins with aromatic groups (AmberLite^{™} FPX66 and Purosorb PAD900) presented the lowest Ct compared to the methods with other resins.

It is important to highlight that since the experimental conditions are the same in all the samples, except for the resin, it can be inferred that the amount of DNA after the thermal process is the same in all the samples, so the difference in Ct it is mainly caused by the number of free inhibitors that affect the RT-PCR and by the amount of free DNA that is available for amplification.

As a result of the above, these results demonstrate that the nucleic acid extraction device comprising a non-ionic adsorption resin with aromatic groups reduces the number of inhibitors contained in the sample and consequently increases the sensitivity of the RT-PCR to detect RNA-like nucleic acids. The methods that used the Purosorb ^{™} PAD900 and AmberLite^{™} FPX66 resins presented significant differences with respect to the other methods. These differences are surprising and show that not any resin, and in particular, only one of the adsorption types with aromatic groups, can increase the sensitivity of the RT-PCR for the detection of SARS-COV-2 RNA.

### Example 2: Comparison of extraction and purification using the device of the invention versus column extraction device. Clinical validation of the device.

To compare the sensitivity of the device of the invention with respect to other commercial kit alternatives available on the market, real-time RT-PCR assays were performed using as template nucleic acid those that were extracted by the device developed in the present invention and others by the EZNA^{®} Total RNA Kit I from Omega Biotek. This is a widely used kit for the extraction and purification of nucleic acids from samples presumed to have the virus, and can be considered as a reference method in the art. Said kit uses purification columns in which a cell lysate of the biological sample to be purified is deposited, the sample is centrifuged, and the RNA of the virus remains adhered to the matrix of the column, passing the inhibitors and other interfering molecules in the eluate.

The extraction and processing of the samples with the device of the invention was identical to that described in Example 1 of the present application. The sample extraction protocol used for the Omega Biotek EZNA^{®} Total RNA Kit I was as described by the manufacturer. Briefly this protocol consists of 6 steps:
add lysis buffer and incubate for 10 minutes;
add bind buffer;
add the sample to the column and then centrifuge;
add wash buffer and centrifuge;
add again the washing buffer and centrifuge, and;
add elution buffer, centrifuge, and elute nucleic acids to a receiving tube.

This entire process takes about 1 hour for 24 samples. It is important to specify that the clinical samples used to carry out these comparative tests were the same. That is, biological samples from nasopharyngeal and/ or oropharyngeal swabs that had previously been determined to be positive for SARS-COV-2 were saved, and a solution of said samples was used as starting material for the detection of nucleic acids. The results obtained by real-time RT-PCR are shown in Table 2.

**Table 2. Comparison of detection sensitivity of SARS-COV-2 by real-time RT-PCR of the device of the present invention with respect to a reference kit.**

| **Sample** | **Ct N1 with kit EZNA^{®} Total RNA Kit I (OMEGA biotek)** | **Ct N1 with device TAAG** | **Differences** |
|---|---|---|---|
| 1 | 23.3 | 22.0 | -1.4 |
| 2 | 27.7 | 27.1 | -0.6 |
| 3 | 27.1 | 24.5 | -2.6 |
| 4 | 31.1 | 30.1 | -1.0 |
| 5 | 23.5 | 21.7 | -1.7 |
| 6 | 24.4 | 20.4 | -3.9 |
| 7 | 25.0 | 24.1 | -1.0 |
| 8 | 23.8 | 23.7 | -0.1 |
| 9 | 21.5 | 19.6 | -1.8 |
| 10 | 29.8 | 28.4 | -1.4 |
| 11 | 34.3 | 32.9 | -1.4 |
| 12 | 29.0 | 27.2 | -1.8 |
| 13 | 26.7 | 25.3 | -1.4 |
| 14 | 28.4 | 27.0 | -1.4 |
| 15 | 24.8 | 24.5 | -0.3 |

As can be seen from Table 2, the nucleic acid extraction and purification device has a sensitivity of more than one Ct difference in 12 of the 15 clinical samples evaluated. Therefore, it can be concluded that the extraction device comprising a non-ionic adsorption resin for extracting nucleic acids from a sample comprising only the steps of contacting the biological sample with the extraction device and heating the sample at 95ºC for 15 min, it is a method that produces RNA that is cleaner from inhibitors and/or a greater amount of RNA available for amplification than the gold standard for SARS-COV-2 RNA extraction currently available in the industry.

It should be noted that this improvement of the device developed in the present invention is unexpected and surprising, since it was not possible to predict that the simple incubation of the biological sample containing a nucleic acid with a non-ionic adsorption resin, and heating the sample for 15 min at 95ºC, would generate improved technical effects compared to the gold standard in SARS-COV-2 RNA extraction, which has a series of purification steps. As an expert in the field will recognize, the developed device will generate a reduction in time and costs in any laboratory that implements this type of simplified extraction device.

### Example 3: Evaluation of the combined effect of non-ionic adsorption resin and heat treatment on detection sensitivity

One of the experimental objectives that guided the development of the present invention was to determine if the combination of the non-ionic adsorption resin with the heat treatment was essential in the effectiveness of the extraction method developed. To determine if both treatments were necessary to achieve the desired effectiveness, an experiment was designed where the treatments were evaluated separately.

Treatment 1 described in the present example refers to incubating a biological mixture comprising the nucleic acid to be detected with the buffer of the extraction device, but the nucleic acid extraction was performed without the resin. That is, the biological sample was placed in contact with the buffer solution, and then it was heated for at least 15 min at 95ºC. The resulting solution was then used as a template for real-time PCR assays as described above.

Treatment 2 described in this example refers to incubating a biological mixture comprising the nucleic acid to be detected with the buffer solution of the extraction device (without the resin) and then incubating said resulting solution for at least 15 min at 95ºC. After said incubation, an appropriate volume of the solid resin as previously described in the present application is added and allowed to incubate for a suitable period of time. Finally, a volume of said solution is taken to be used as a template for real-time PCR assays.

Treatment 3 described in this example refers to the normal nucleic acid extraction and purification method that comprises simultaneous incubation of the resin with the biological sample and then a heat treatment for at least 15 min at 95°C. This treatment corresponds to the control of the experiment.

Table 3 shows the results obtained from real-time tests for each of the treatments. It should be noted that, as indicated for example 2, each of the samples evaluated had been previously determined to be positive for SARS-COV-2.

**Table 3. Effect of resin and heat treatment on device detection sensitivity.**

| **Sample** | **Ct N1 with treatment 1** | **Ct N1 with treatment 2** | **Ct N1 with treatment 3** |
|---|---|---|---|
| **Negative** | 40 | 40 | 40 |
| **Sample 1** | 30.16 | 30.03 | 23.37 |
| **Sample 2** | 25.87 | 25.56 | 22.41 |
| **Sample 3** | 23.11 | 23.07 | 21.54 |
| **Sample 4** | 22.77 | 22.45 | 21.45 |
| **Sample 5** | 24.04 | 24.02 | 22.43 |

The results shown in Table 3 show that treatment 3 is the most sensitive for the detection of SARS-CoV-2. No significant differences were observed in terms of the sensitivity observed between treatments 1 and 2.

These results clearly demonstrate that the resin together with the heat treatment works better than both alone. These results are unexpected and surprising, since the protocols of the most widely used methods to extract nucleic acids have at least two clearly differentiated steps: Lysis and Purification, while the device developed in the present invention unifies these two steps, generating a protocol simpler and faster.

### Example 4: Comparison of extraction and purification using the device of the invention by varying the heat treatment.

In a further embodiment, the effect of temperature and time of heat treatment on the sensitivity of the method was evaluated. Table 4 shows different heat treatment regimens to which different samples positive for SARS-COV-2 were subjected.

**Table 4. Effect of different temperatures and incubation times on detection sensitivity.**

| **Sample** | **Treatment** | |
|---|---|---|
| | **Ct N1 with 95°C x 15'** | **Ct N1 with 95°C x 30'** |
| **1** | 18.84 | 18.80 |
| **2** | 31.63 | 31.98 |
| **3** | 26.38 | 26,26 |
| **4** | 24.72 | 24.79 |
| **5** | 22.96 | 23.11 |
| **6** | 25.92 | 26,27 |

As can be seen in said table, there are no significant differences in terms of the sensitivity of the detection of SARS-CoV-2 when the incubation time was varied (15 min or 30 min).

It is important to mention that in this example both conditions have an incubation of 15 minutes at 95ºC. This stage is necessary to inactivate SARS-CoV-2 according to WHO guidelines, which is why it is the minimum temperature and time used. However, as one skilled in the art will recognize, lower temperature and shorter time could be just as efficient in releasing nucleic acids from microorganisms.

### Example 5: Comparison of extraction and purification using the device of the invention by varying the proportions of resin and buffer solution.

Another of the preferred embodiments of the present invention was to search for the optimal ratio between the amount of resin and the amount of a solution comprising the biological sample and the buffer solution specifically for the detection of SARS-CoV-2. For this, the biological sample in the form of a nasopharyngeal swab was deposited in a first container containing an appropriate amount of buffer solution. The mixture was homogenized and then different volumes of said mixture were taken and mixed each with 0.06 grams of solid resin deposited in a separate container. Then, the samples were incubated for 15 min at 95ºC, and real-time RT-PCR assays were performed as previously described.

Table 5 shows the results of these tests for each of the proportions resin: buffer solution + evaluated sample.

**Table 5. Effect of the ratio of the amount of resin versus the amount of buffer solution and sample on the sensitivity of the device.**

| **Condition** | **Ct N1** |
|---|---|
| Control: 20µl sample + buffer | 25.22 |
| 0.06 grams resin: 20µl sample + buffer | 25.43 |
| 0.06 grams resin: 40µl sample + buffer | 23.88 |
| 0.06 grams resin: 80µl sample + buffer | 23.11 |
| 0.06 grams resin: 120µl sample + buffer | 22.16 |
| 0.06 grams resin: 150µl sample + buffer | 22.85 |
| 0.06 grams resin: 200µl sample + buffer | 22.54 |

From the above table it can be seen that there is a specific proportion of resin and buffer mixture and sample, which maximizes the sensitivity of the method. The optimal proportion of resin and sample plus buffer solution, for the case of nasopharyngeal swab samples from patients positive for SARS-CoV-2, was 0.06 grams of resin and 120 µL of sample plus buffer solution.

**These** results suggest that there is a careful chemical balance between the different components present in a certain biological sample, and these tests show that there must be an adequate proportion to maximize the results that are desired.

It is important to mention that this example intends to demonstrate the importance of the proportions of resin versus sample and buffer to optimize the extraction of SARS-CoV-2 RNA. For other applications these ratios could vary, as shown in Example 6.

### Example 6: Validation from previously spiked food samples

As mentioned above, another of the preferred embodiments of the invention is the extraction and purification from different food samples and/or surfaces. To evaluate whether the device of the present invention was capable of extracting and purifying nucleic acids from microorganisms in such samples, the device was used in different matrices.

Table 6 shows the results obtained in the different samples.

**Table 6. Detection of nucleic acids from microorganisms in various matrices.**

| Matrix | *S. aureus* | | *L. monocytogenes* | | *S. enteric* | | *E. coli* | |
|---|---|---|---|---|---|---|---|---|
| | inoculated | detect | inoculated | detect | inoculated | detect | inoculated | detect |
| Manipulator | x | x | x | x | x | x | x | x |
| Manipulator | x | x | x | x | x | x | x | x |
| Manipulator | x | x | x | x | x | x | x | x |
| Surface | x | x | x | x | x | x | x | x |
| Surface | x | x | x | x | x | x | x | x |
| Surface | x | x | x | x | x | x | x | x |
| Breast ofTurkey | x | x | x | x | x | x | x | x |
| Seasoning | x | x | x | x | x | x | x | x |

As can be seen from the table above, the nucleic acid extraction device is capable of detecting the four microorganisms evaluated in each of said matrices. This is particularly important in the seasoning sample, which are samples known to have PCR inhibitors.

In this example, the enriched samples were added directly onto tubes with resin, without any type of buffer, and the results were satisfactory. As a result of the foregoing, it can be deduced that, depending on the application, the device developed in the present invention may or may not contain a buffer solution.

### Example 7: Comparison of extraction and purification using the device of the invention versus column extraction device. Clinical validation of the device in saliva samples.

To compare the efficiency of the device of the invention with respect to other commercial kit alternatives available on the market for the extraction of RNA from saliva, real-time RT-PCR assays were performed using as template nucleic acid those that were extracted by the device developed in the present invention and by others by the EZNA Total RNA Kit I from Omega Biotek.

It is important to specify that the clinical samples used to carry out these comparative tests were the same. That is, biological saliva samples that had been previously determined to be positive for SARS-COV2 were saved, and a solution of said samples was used as starting material for the detection of nucleic acids. The results obtained by real-time RT-PCR are shown in Table 7.

**Table 7. Comparison of the detection sensitivity of SARS-COV2 by real-time RT-PCR of the device of the present invention in saliva samples with respect to a reference kit.**

| **Sample** | **Ct N1 with kit E.Z.N.A.^{®} Total RNA Kit I (OMEGA BioTek)** | **Ct N1 with device TAAG** | **Differences** |
|---|---|---|---|
| 1 | 27.55 | 27.96 | 0.41 |
| 2 | 28.36 | 27.54 | -0.82 |
| 3 | 29.84 | 27.73 | -2.11 |
| 4 | 26.94 | 27.39 | 0.45 |
| 5 | 29.21 | 27.06 | -2.15 |
| 6 | 28.13 | 27.38 | 0.75 |
| 7 | 27.19 | 26.15 | -1.04 |
| 8 | 27.46 | 26.88 | -0.58 |
| 9 | 27.34 | 26.38 | -0,96 |
| **Mean** | **28.00** | **27.16** | **-0.84** |

**As** can be seen from Table 7, the saliva nucleic acid extraction and purification device generally performs better than the Gold Standard for SARS-COV2 RNA extraction currently available in the industry.

### Example 8: Comparison of extraction and purification using the device of the invention by varying the temperature of the heat treatment.

In a further embodiment, the effect of different temperatures on the sensitivity of the method was evaluated. Table 8 shows the efficiency of the method according to different incubation temperatures to which a positive sample for SARS-COV2 was subjected.

**Table 8. Effects of different temperatures on detection sensitivity.**

| **Temperature °C x15 min.** | **Ct N1** |
|---|---|
| 95.0 | 25.59 |
| 93.7 | 25.80 |
| 90.9 | 24.75 |
| 86.1 | 26.11 |
| 80.4 | 26.33 |
| 75.8 | 26.78 |
| 72.6 | 28.19 |
| 71.0 | 28.50 |

As observed in said table, temperatures above 75°C maximize the efficiency of the method. The optimal temperature will depend on additional sample treatment requirements, which in the case of SARS-COV2 must also be considered for inactivation.

### Example 9: Comparison of extraction and purification using the device of the invention by varying the heat treatment.

In a further embodiment, the effect of heat treatment time on the efficiency of the method was evaluated. Table 9 shows the results obtained after incubating at 95°C, with different incubation times, a positive sample for SARS-COV2.

**Table 9. Effect of different times on detection sensitivity.**

| **Time (min.)** | **Ct N1** |
|---|---|
| **0 (no resin)** | 25.59 |
| **0 (no resin)** | 25.80 |
| **1 (with resin)** | 24.75 |
| **5 (with resin)** | 26.11 |
| **15 (with resin)** | 26.33 |
| **45 (with resin)** | 26.78 |
| **90 (with resin)** | 28.19 |

As seen in Table 9, incubating the samples at high temperature has a very important effect on the efficiency of nucleic acid extraction.

It is important to mention that the samples were analyzed to detect the presence of the SARS-COV2 virus, so a very short incubation period was enough to release the genetic material of the virus, however, for more complex microorganisms such as bacteria and fungi, this time incubation time could be longer to achieve efficient release of genetic material.

### Example 10: Comparison of extraction and purification using the device of the invention, varying the type of resin.

The effect of different types of ionic resins and non-ionic aromatic and/or aliphatic absorbent resins on the sensitivity of the method was evaluated. The real-time RT-PCR results for a SARS-COV2 positive sample processed with different resins are shown in Figure 1.

As observed in Fig.1, non-ionic aromatic and/or aliphatic absorbent resins maximize the sensitivity of the method compared to the use of ionic resins.

### INDUSTRIAL APPLICATION

The present invention has a wide application in the biotechnological, biomedical and food industry. Likewise, the present invention provides an innovative application for the rapid and effective extraction of nucleic acids from different samples.

## Claims

1. A device for the extraction and purification of nucleic acids, **characterized in that** the device comprises a closed container that allows heat treatment of a mixture that includes a solid phase composed of non-ionic aliphatic and/or aromatic adsorbent resins, an aqueous solution and a sample of biological origin containing the nucleic acids, which after heating, allows the detection of nucleic acids.

2. A device according to claim 1, **characterized in that** device allows the direct detection of nucleic acids without the need to separate the solid and liquid phases.

3. The device according to claims 1 and 2, **characterized in that** the heat treatment includes heating the sample at a temperature between 50ºC to 100ºC for 1 to 30 minutes.

4. The device according to claim 3, **characterized in that** the heating temperature is preferably at 95°C for at least 15 minutes.

5. The device according to any of claims 1 to 4, **characterized in that** the solid phase is an aromatic and/or aliphatic adsorbent polymeric resin added to the container in a proportion with respect to the other components from 5% to 300% w/v, preferably from 30% to 150% w/v, and more preferably from 50% to 100% w/v.

6. The device according to any of claims 1 to 5, **characterized in that** the non-ionic aliphatic and/or aromatic adsorbent resin is selected from PuroSorb^{®} PAD400, PAD500, PAD600, PAD900, PAD1200, PAD350, PAD610, PAD910, PAD950, PAD950C, Amberlite^{®} FPX66, FPX68, Amberlite^{®} XAD2, XAD4, XAD16, XAD1180, XAD200, XAD2010, XAD16N, XAD1600N, XAD18, XAD1180N, XAD7HP, XAD761, Diaion^{®} and Sepabeads^{®} HP20, HP20SS, HP21, SP70, SP700, SP825L, SP850, CHP20, CHP50, SP207, HP2MGL, LEWATIT^{®} AF 5, SEPLlTE^{®} CT10, LX20, LX207, LXA8, LXA10, LXA17, LXA680, LXA1600, LXA1180, LXA81, LXA816, LXA817, LXA8302, LXA88, LXS868 and AB-8 or other aromatic or aliphatic resins.

7. The device according to any of claims 1 to 6, **characterized in that** the nonionic solid matrix may comprise a mixture of nonionic aliphatic and/or aromatic adsorbent resins.

8. The device according to any of claims 1 to 7, **characterized in that** the nonionic solid matrix is a mixture between different nonionic aliphatic and/or aromatic adsorbent resins.

9. The device according to any of the claims 1 to 8, **characterized in that** the amount of the non-ionic aliphatic and/or aromatic adsorbent resin in a container is 0.6 grams of resin mixed with 1 mL of an aqueous buffer solution.

10. The device according to any of claims 1 to 9, **characterized in that** the liquid that is added to the resin is selected from: (a) a buffer solution that can act to maintain the pH of the mixture at acidic, neutral and basic pH; and (b) water.

11. The device according to any of claims 1 to 10, **characterized in that** the buffer solution is selected from citric acid, phosphate, MES, Bis-Tris, ADA, ACES, PIPES, MOPSO, Bis-Tris Propane, BES, MOPS, TES, HEPES, DIPSO, TAPSO, Trizma, HEPPSO, POPSO, TEA, EPPS, Tricine, Gly-Gly, Bicine, HEPBS, TAPS, AMPD, TABS, AMPSO, CHES, CAPSO, AMP, CAPS and/or CABS.

12. The device according to any of claims 1 to 11, **characterized in that** the biological sample is selected from samples of animal, plant, and microbiological origin that contain cells, bacteria, or viral particles whose nucleic acids are to be detected, and wherein said samples contain inhibitors that interfere with the detection of nucleic acids.

13. The device according to any of claims 1 to 12, **characterized in that** samples include bacterial and fungi and yeast spikes, and human biological samples for the detection of bacteria and viruses.

14. The device according to any of claims 1 to 13, **characterized in that** the human biological sample comes from a nasopharyngeal swab, oropharyngeal swab, nasal swab, oral swab, vaginal swab, cervical swab, urethral swab, saliva, dermatological samples, blood and plasma.

15. The device according to any of claims 1 to 14, **characterized in that** the container corresponds to a tube that allows the biological sample to be deposited, transported safely, and heat treated.

16. The device according to any of claims 1 to 15, **characterized in that** the container is selected from plastic tubes, including 50 mL or 15 mL Falcon types, 2 mL, 1.5 mL, 1 mL, or 0.5 mL Eppendorf tubes, PCR tubes, 96-well plates, and 384-well plates.

17. The device according to any of claims 1 to 16, **characterized in that** the non ionica aliphatic and/or aromatic adsorbent resins can be used in column, filter or disk purification devices.

18. A method of extraction and purification of nucleic acids from biological samples using the devices according to claims 1 to 17, **characterized in that** consists of the following steps:
a) provide a sample of biological origin;
b) deposit the sample of biological origin containing the nucleic acids in the closed container;
c) heat the sample;
d) take a volume of the sample, and
e) detect the presence of the nucleic acid of interest by means of PCR or any of its variants.

19. The extraction method according to claim 18, **characterized in that** the sample is heated by incubation at 95ºC for at least 15 min.

20. The extraction method according to claim 19, **characterized in that** the nucleic acid detection is performed by real-time PCR, RT-PCR, Multiplex PCR, nested PCR and Hot start PCR.
